# EUROPÄISCHE PATENTSCHRIFT

(11) **EP 0 915 681 B1**
(45) Veröffentlichungstag und Bekanntmachung des Hinweises auf die Patenterteilung: **13.12.2000**
(21) Anmeldenummer: 98912463.1
(22) Anmeldetag: 11.03.1998
(51) Int. Cl.: A61B 17/34

(54) **Rohrschaftinstrument, insbesondere Trokar**
Trocar-like tubular instrument
Instrument tubulaire du type trocart

(30) Priorität: 26.03.1997 DE 19712726
(43) Veröffentlichungstag der Anmeldung: 19.05.1999
(73) Patentinhaber: Karl Storz GmbH & Co. KG, 78532 Tuttlingen (DE)
(72) Erfinder: DITTRICH, Horst, D-78194 Immendingen (DE); BACHER, Uwe, D-78532 Tuttlingen (DE)
(74) Vertreter: Weller, Wolfgang, Dr.rer.nat.
(86) Internationale Anmeldenummer: EP9801389
(87) Internationale Veröffentlichungsnummer: WO9842265

(56) Entgegenhaltungen:
- EP-A- 0 267 584
- DE-A- 3 923 243
- DE-U- 9 418 005

## Beschreibung

Die Erfindung betrifft ein Rohrschaftinstrument, insbesondere einen Trokar, mit einem Instrumentenkanal, der durch ein Klappventil verschließbar ist, sowie mit einem Betätigungselement, um das Klappventil gegen die Kraft einer Schließfeder zu öffnen.

Ein derartiges Rohrschaftinstrument in Form eines Trokars ist aus der DE 39 23 243 C2 bekannt.

Das Klappventil enthält eine Klappe, die in geschlossenem Zustand sich quer zum Instrumentenkanal erstreckt. Die Klappe ist um eine neben dem Instrumentenkanal gelegene Schwenkachse verschwenkbar und wird durch die Kraft einer Schließfeder in Schließrichtung gedrückt.

Das Betätigungselement zum Öffnen der Klappe besteht aus einer Ringscheibe, die den Instrumentenkanal umrundet und die axial in Richtung der Instrumentenkanalachse verschiebbar ist. Die Scheibe steht über einen sich längs der Instrumentenkanalachse erstreckenden Stößel mit der Klappe des Klappventils in Verbindung. Der Stößel ist neben dem Instrumentenkanal angeordnet und trifft in einem Bereich benachbart zur Schwenkachse bzw. der Scharnierachse, an die die Klappe angelenkt ist, auf die geschlossene Klappe. Zum Öffnen des Klappventils wird das Betätigungselement axial verschoben, dabei wird der Stößel ebenfalls axial verschoben und stößt die Klappe auf. Der Instrumentenkanal ist nunmehr offen, so daß ein Instrument, bspw. ein Trokardorn oder ein Endoskop, durch den Trokar hindurchgeschoben werden kann. Eine Feder drückt das freigegebene Betätigungselement wieder in dessen Ausgangsstellung zurück, die Klappe verschwenkt dann soweit in Schließrichtung, bis diese auf das eingeschobene Instrument trifft. Je nach Ausgestaltung der Außenseite des Instrumentes kann dieses einfach abgezogen werden, wonach die Klappe aufgrund der Schließfeder wieder in die Schließstellung gedrückt wird und den Instrumentenkanal verschließt. Dadurch können bspw. beim Instrumentenwechsel keine Gase oder Flüssigkeiten über den Instrumentenkanal aus dem Körper, in den der Trokar eingeführt ist, austreten. Sollte die am Instrument anliegende Klappe zum Abziehen des Instrumentes zunächst von diesem weg bewegt werden, wird das Betätigungselement wieder axial verschoben und die Klappe wird über den Stößel wieder in ihre maximale Offenstellung verschwenkt.

Diese Konstruktion benötigt einen relativ großen Bauraum neben dem eigentlichen Instrumentenkanal und führt bei kleinen Trokaren, bspw. bei Kindertrokaren, zu unnötig ausladenden Baugrößen. Bei manchen Rohrschaftinstrumenten ist es ferner erwünscht, bezüglich des nutzbaren lichten Innendurchmessers des Instrumentenkanals möglichst wenig radiale Baubreite notwendig zu machen.

Aus der DE 70 04 051 U1 ist eine Trokarhülse bekannt, deren Instrumentenkanal ebenfalls über ein Klappventil verschließbar ist.

Zum Öffnen der Klappe ist ein begrenzt verschiebbar gelagerter Zylinder vorgesehen, der mit seiner einen ringförmigen Stirnfläche auf der geschlossenen Klappe ruht. Im Innern des Zylinders ist ein geschlitzter Federring aus Kunststoff eingesetzt.

Wird nunmehr ein Trokardorn in die Trokarhülse eingesetzt, so trifft dessen Spitze auf den geschlitzten Federring, dringt teilweise durch diesen hindurch, weitet diesen dabei etwas auf, bis ein solcher Reibschluß geschaffen ist, daß ein weiteres Vorantreiben des Trokardorns den verschiebbar gelagerten Zylinder, der den geschlitzten Federring trägt, distal verschiebt. Dabei stößt das distale Ende des Zylinders die Klappe des Klappventils auf. Dadurch, daß der Zylinder nur eine begrenzte Wegstrecke voranschiebbar ist, trifft dieser auf einen Anschlag, so daß ein weiteres Vorantreiben des Trokardorns dazu führt, daß der geschlitzte Federring so weit geöffnet wird, daß der Trokardorn weiter im Instrumentenkanal distal verschoben werden kann. Beim Abziehen nimmt der Trokardorn den Zylinder wieder soweit zurück, bis er an einen Anschlag trifft. Dadurch kann die federbelastete Klappe wieder einschwenken und den Instrumentenkanal verschließen.

Diese Konstruktion erfordert exakt auf den Zylinder abgestimmte Instrumente, um für den für den Bewegungsablauf notwendigen Reibschluß zu sorgen. Ist der Reibschluß zwischen Instrument und geschlitztem Federring nicht ausreichend, kann das Instrument durch den Zylinder hindurchtreten, ohne daß dieser verschoben wird, und somit nicht die Klappe öffnet, sondern dies muß dann durch das eingeschobene Instrument erfolgen, was bei einem Trokardorn, der sehr spitz ausgeführt ist, zu Beschädigungen bzw. zum Verkanten oder Verklemmen führt. Ist das Instrument aufgrund von Fertigungstoleranzen zu groß, findet ein Verklemmen zwischen dem Instrument und dem Zylinder statt, so daß der Bewegungsablauf behindert ist.

Es ist Aufgabe der vorliegenden Erfindung, ein Rohrschaftinstrument der eingangs genannten Art, vgl. DE-A- 3923243, derart weiterzuentwikkeln, daß mit konstruktiv einfachen und wenig raumergreifenden Mitteln ein handhabungsfreundliches Steuern der Öffnungs- bzw. Schließbewegung des Klappventils möglich ist.

Erfindungsgemäß wird die Aufgabe dadurch gelöst, daß das Betätigungselement als ein den Instrumentenkanal zumindest teilweise umrundendes, um die Instrumentenkanalachse verdrehbares Elementes ausgebildet ist, das mit einer umfänglichen, in einer Richtung parallel zur Instrumentenkanalachse ansteigenden Anlaufschräge versehen ist, auf der ein Steuerabschnitt des Klappventils ruht, so daß ein Verdrehen des verdrehbaren Elementes ein Öffnen bzw. ein Schließen des Klappventils bewirkt.

Die Maßnahme, daß ein um die Instrumentenkanalachse verdrehbares Element mit Anlaufschräge eingesetzt wird, hat den Vorteil, daß keine weit abseits des Instrumentenkanals angeordnete Bauelemente, wie bspw. der eingangs erwähnte Stößel vorhanden sind, sondern daß die zur Steuerung notwendigen Elemente direkt um den Instrumentenkanal herum angeordnet sind.

Die Drehbewegung beim Steuern der Öffnungs- bzw. Schließstellung des Klappventils anstatt einer axial gerichteten Bewegung hat den Vorteil, daß auf das Rohrschaftinstrument keine Kräfte einwirken, die diesen in Richtung Körperinnerem verschieben. Insbesondere bei der minimalinvasiven Chirurgie werden im Laufe einer Operation mehrere unterschiedliche Instrumente in einen Trokar eingesetzt, der währenddessen im Körper des Patienten verbleibt. Die Drehbewegung hat keinen Einfluß auf die Einsetztiefe des Trokars.

Durch das Vorsehen einer in Richtung der Instrumentenkanalachse gesehen dagegen ansteigenden Anlaufschräge, auf der ein Steuerabschnitt des Klappventils ruht, wird ermöglicht, nunmehr die Drehbewegung des verdrehbaren Elementes in eine öffnende bzw. schließende Verschwenkbewegung der Klappe des Klappventiles umzusetzen.

Steigt die Anlaufschräge bspw. von distal nach proximal gesehen an und soll die Klappe aus einer geschlossenen Stellung, in der diese sich quer zur Längsrichtung des Instrumentenkanals erstreckt, nach distal verschwenkt werden, steht der Steuerabschnitt des Klappventils mit dem höchsten (proximalsten) Ende der Anlaufschräge in Berührung. Durch Verdrehen des verdrehbaren Elementes wird der Steuerabschnitt und somit das Klappventil nach unten (nach distal) verschwenkt und somit geöffnet. Die Bewegung erfolgt sinnvollerweise soweit, bis das Klappventil gerade aus dem Instrumentenkanal heraus verschwenkt ist, so daß dieser in voller Breite für das einzuschiebende Instrument zur Verfügung steht. In dieser Stellung steht der Steuerabschnitt mit dem untersten (distalen) Ende der Anlaufschräge in Berührung.

Durch Drehen des verdrehbaren Elementes in die entgegengesetzte Richtung kann der Steuerabschnitt von unten (distal) nach oben (proximal) längs der Anlaufschräge laufen, wobei die Klappe nach innen verschwenkt und den Instrumentenkanal verschließt.

Soll die Klappe nach proximal verschwenkt werden, ist der Anstieg der Anlaufschrägen umgekehrt.

Diese Bewegung wird durch die Kraft der Schließfeder, die auf die Klappe in Schließrichtung wirkt, unterstützt.

Das den Instrumentenkanal zumindest teilweise umrundende verdrehbare Element kann von der Außenseite einfach durch eine Hand ergriffen und verdreht werden. Aus Stabilitätsgründen wird das verdrehbare Element den Instrumentenkanal meist vollständig umrunden, es reicht aber auch aus, wenn der Instrumentenkanal nur zum Teil, z.B. halb- oder dreiviertelkreisförmig umrundet ist.

Somit wird die Aufgabe vollkommen gelöst.

In einer weiteren Ausgestaltung der Erfindung weist das verdrehbare Element zwei gegenüberliegende gleichsinnige Anlaufschrägen auf, so daß ein Drehen des verdrehbaren Elementes in die eine oder in die entgegengesetzte Richtung jeweils dieselbe Bewegung des Klappventiles verursacht.

Diese Maßnahme hat nun den erheblichen Vorteil, daß unabhängig von der Drehrichtung durch die beiden gleichsinnigen etwa diametral gegenüberliegenden Anlaufschrägen das Klappventil geöffnet werden kann. Der Steuerabschnitt des Klappventiles ist dann so ausgebildet, daß er mit beiden Anlaufschrägen in Berührung steht, so daß unabhängig davon, ob das verdrehbare Element in der einen oder in der entgegengesetzten Richtung gedreht wird, jedesmal die Klappe geöffnet wird. Dies hat den erheblichen handhabungsfreundlichen Vorteil, daß die Bedienungsperson sich keine bestimmte Drehrichtung zum Öffnen der Klappe einprägen muß, sondern daß dies in beiden möglichen Drehrichtungen erfolgen kann.

In einer weiteren Ausgestaltung der Erfindung ist das verdrehbare Element als Hülse ausgebildet, deren dem Klappventil zugewandtes Ende schräg abgeschnitten ist, wobei die Schnittkanten die Anlaufschrägen bilden.

Diese Maßnahme hat nun den konstruktiven und fertigungstechnischen Vorteil, daß durch einfache Maßnahmen ein radial nur schmal bauendes verdrehbares Element geschaffen wird, dessen Ende schräg abgeschnitten ist, wodurch dann die Anlaufschrägen gebildet sind.

In einer weiteren Ausgestaltung der Erfindung ist das Ende des verdrehbaren Elementes etwa über einen halben Umfang schräg abgeschnitten.

Diese Maßnahme hat den Vorteil, daß ein Drehen um 90° in der einen oder in der anderen Richtung jeweils ein vollständiges Öffnen der Ventilklappe bewirkt. Gleichzeitig ist es in dem abgeschnittenen Abschnitt möglich, daß die eingeschwenkte, also geschlossene Ventilklappe in den Innenraum einschwenkt, um den Instrumentenkanal zu verschließen. Im verschlossenen Zustand ist also die Klappe über den abgeschnittenen Bereich in den Endabschnitt des verdrehbaren Elementes bzw. der Hülse eingetreten. In diesem Zustand ist dann die Klappe durch die etwa diese um einen halben Umfang umgebenden nicht abgeschnittenen Bereiche geschützt, so daß beim Zerlegen oder Montieren keine Beschädigungen an der geschlossenen Ventilklappe zu befürchten sind.

In einer weiteren Ausgestaltung der Erfindung verläuft der Schnittwinkel etwa unter 45° zur Instrumentenkanalachse.

Diese Geometrie erlaubt eine günstige Kraftübertragung von dem sich verdrehenden Element auf das Klappventil, ohne daß am Anfang oder am Ende große Kraftspitzen notwendig sind, so daß eine gleichmäßige und harmonische Öffnungsbewegung sichergestellt ist, was sehr handhabungsfreundlich ist.

In einer weiteren Ausgestaltung der Erfindung ist das verdrehbare Element mit zumindest einem radial abstehenden Griffelement versehen.

Diese Maßnahme hat den Vorteil, daß durch das radial abstehende Griffelement eine Hebelwirkung erzielt wird, so daß eine relativ geringe Kraft notwendig ist, um die Klappe zu öffnen. In Kombination mit der beidseitigen Verschwenkbarkeit ist es besonders vorteilhaft, d.h. es muß mit dem Finger lediglich das radial abstehende Griffelement ergriffen oder der Finger an dieses angelegt und dann in die eine oder andere Drehrichtung verschwenkt werden.

In einer weiteren Ausgestaltung der Erfindung sind zwei diametral gegenüberliegende, radial abstehende Griffelement vorgesehen.

Diese Maßnahme hat den Vorteil, daß mehrere günstige Anlegepositionen für den Finger einer Hand vorgesehen sind, um mit einem geringen Kraftaufwand das verdrehbare Element zu verdrehen, um dadurch die Ventilklappe zu öffnen oder zu schließen.

In einer weiteren Ausgestaltung der Erfindung ist das verdrehbare Element gegen die Kraft einer Torsionsfeder verdrehbar, die das verdrehbare Element in eine Drehstellung dreht, in der das Klappventil geschlossen ist.

Diese Maßnahme hat nunmehr den Vorteil, daß, falls bspw. das verdrehbare Element so gedreht wird, daß die Klappe dabei geöffnet wird, das verdrehbare Element nicht mehr weiter bewegt werden muß, sondern von der Torsionsfeder selbst in dessen Ausgangsstellung zurückbewegt wird. Wird bspw. ein Instrument abgezogen, bewirken die Kräfte der Federn, nämlich die Feder, die die Klappe in Schließrichtung drückt und die Torsionsfeder, daß der Steuerabschnitt selbsttätig längs der Anlaufschräge bzw. Anlaufschrägen in die Schließstellung läuft, wobei das verdrehbare Element entsprechend automatisch verdreht wird. Auch diese Maßnahme ist besonders handhabungsfreundlich und stellt ferner sicher, daß bei einem abgezogenen Instrument der Instrumentenkanal auf jeden Fall durch das Klappventil verschlossen ist, so daß keine Gase oder Flüssigkeiten in unerwünschter Weise durch den Instrumentenkanal hindurchtreten können.

In einer weiteren Ausgestaltung der Erfindung ist die Torsionsfeder in einem Ringraum zwischen Außenseite des verdrehbaren Elementes und einer Innenseite eines das Element umschließenden Gehäuses angeordnet.

Diese Maßnahme führt ebenfalls zu einer schlanken Bauweise, da auch die Torsionsfeder nun in einem ringförmigen Raum angeordnet ist, der keine radial ausladenden Baugrößen erfordert.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: schematisiert eine perspektivische Ansicht eines Trokars, von distal nach proximal gesehen, mit geöffnetem Klappventil;
- Fig. 2: eine perspektivische, teilweise aufgeschnittene Ansicht des Trokars von Fig. 1 in nahezu geschlossenem Zustand des Klappventils;
- Fig. 3: einen Schnitt längs der Linie III-III in Fig. 2, wobei das Klappventil in vollkommen geschlossenem Zustand gezeigt ist;
- Fig. 3a: einen unteren Abschnitt der Darstellung von Fig. 3 in einem teilweise geöffneten Zustand des Klappventils;
- Fig. 3b: eine der Darstellung von Fig. 3a entsprechende Darstellung mit vollständig geöffnetem Klappventil;
- Fig. 4: eine stark vergrößerte schematische Darstellung des Zusammenwirkens eines Betätigungselementes mit dem Klappventil, noch in geschlossenem Zustand des Klappventils; und
- Fig. 5: eine der Fig. 4 entsprechende Darstellung in einem teilweise geöffneten Zustand des Klappventils.

Ein in den Figuren dargestelltes Rohrschaftinstrument ist in seiner Gesamtheit mit der Bezugsziffer 10 bezeichnet.

Das Rohrschaftinstrument 10 im dargestellten Ausführungsbeispiel ist als Trokar 12 ausgebildet.

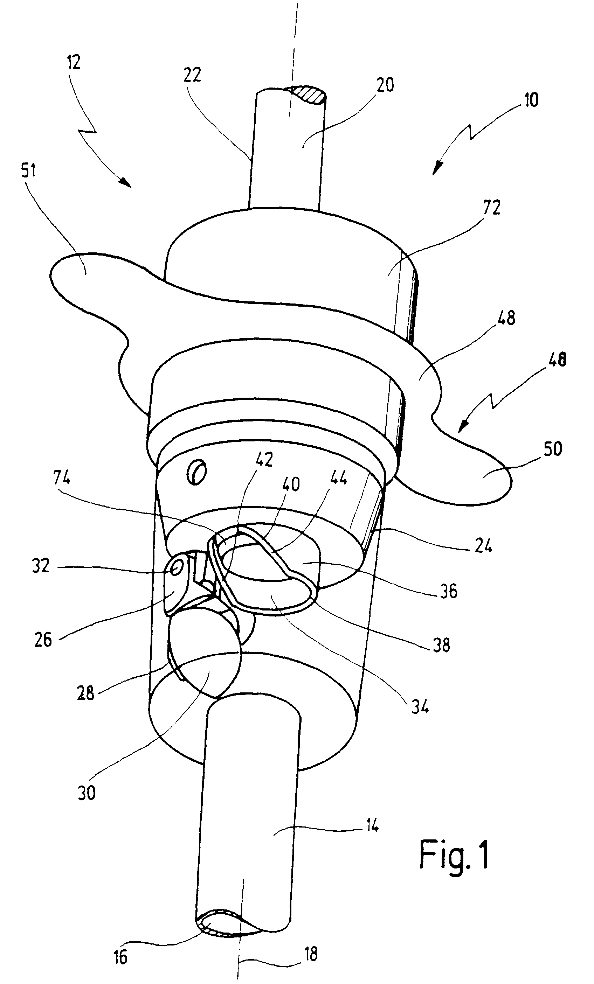

Der in Fig. 1 ersichtliche Trokar 12 weist eine Trokarhülse 14 auf, die einen Instrumentenkanal 16 umrundet. Eine Mittellängsachse des Instrumentenkanals ist als Instrumentenkanalachse 18 bezeichnet. In die Hülse 14 des Trokars 12 soll ein Instrument 20, bspw. ein Trokardorn 22, vom proximalen Ende her eingeführt werden.

Am proximalen Ende der Trokarhülse 14 ist ein Gehäuse 24 vorgesehen, in dessen Innerem ein Klappventil 26 vorgesehen ist.

Das Klappventil 26 weist eine verschwenkbare Klappe 28 auf, die eine Dichthalbkugel 30 trägt.

Die Klappe 28 ist um einen Achszapfen 32 schwenkbar am Gehäuse 24 angelenkt.

Der Achszapfen 32 erstreckt sich seitlich neben dem Instrumentenkanal 16 und quer zur Instrumentenkanalachse 18.

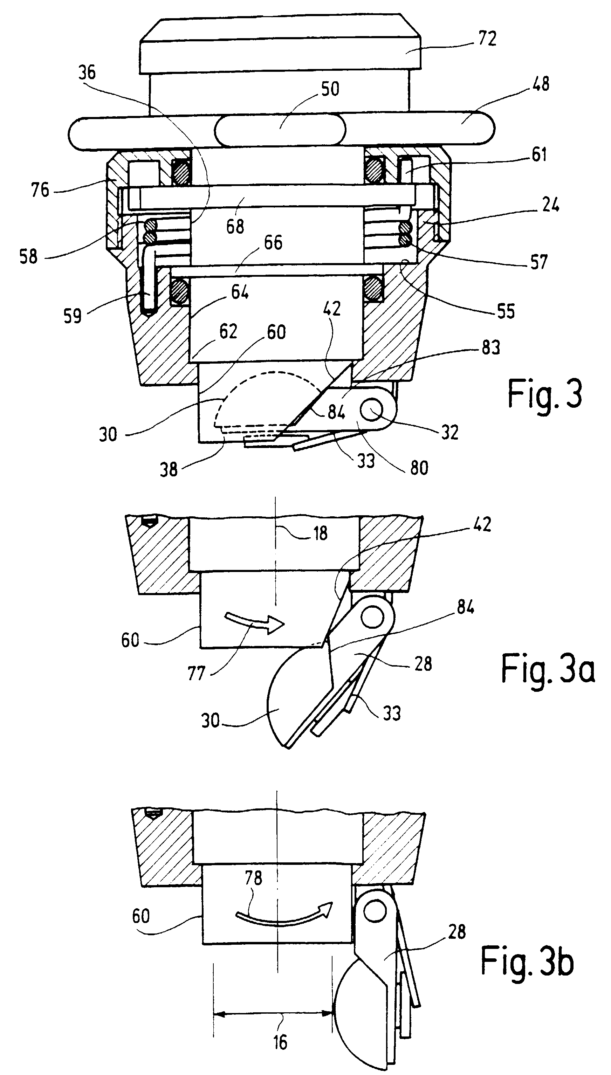

Eine Feder 33 (siehe Fig. 3) wirkt auf die Klappe 28 ein, und zwar derart, daß diese in ihre Schließrichtung bewegt wird.

In der Darstellung von Fig. 1 ist das Klappventil 26 vollständig geöffnet, somit ist der Instrumentenkanal 16 freigegeben, so daß bspw. der Trokardorn 22 durch den Trokar 12 hindurchgeschoben werden kann.

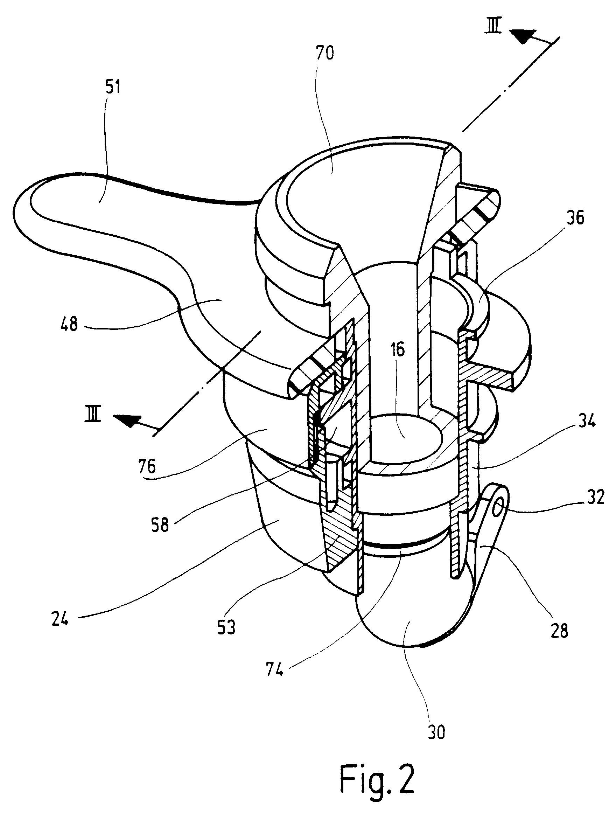

Im Gehäuse 24 ist ein verdrehbares Element 34 in Form einer Hülse 36 aufgenommen, wie das insbesondere aus den Schnittdarstellungen von Fig. 2 und 3 besser zu entnehmen ist, wobei in den Darstellungen von Fig. 3 die Hülse 36 nur angedeutet ist.

Die Hülse 36 ist so im Gehäuse 24 aufgenommen, daß diese den Instrumentenkanal 16 umrundet.

Wie aus der Darstellung von Fig. 1 zu entnehmen ist, ragt ein unteres Ende 38 der Hülse 36 vom Gehäuse 24 nach distal vor. Unmittelbar daneben ist die Klappe 28 über den Achszapfen 32 angelenkt.

Das untere Ende 38 der Hülse 36 ist mit einer schräg verlaufenden Schnittkante 40 versehen, d.h. bis etwa auf den halben Umfang ist das Ende 38 unter einem Winkel vom etwa 45° schräg abgeschnitten.

Dadurch entstehen etwa diametral gegenüberliegend zwei gleichsinnige Anlaufschrägen 42 und 44, die, in der Darstellung von Fig. 1, jeweils vom unteren Ende 38 nach proximal ansteigen.

An dem dem Ende 38 gegenüberliegenden Ende ist die Hülse 36 über ein Aufsatzstück 76 mit einem Griff 46 verbunden, wie das insbesondere aus der Schnittdarstellung von Fig. 2 ersichtlich ist. Der Griff 46 weist einen die Hülse 36 umrundenden Ring 48 auf, von dem zwei diametral gegenüberliegende Griffelemente 50, 51 radial vorspringen.

In einer inneren Wand 53 des Gehäuses 24 ist eine Aussparung 55 vorgesehen, wie das insbesondere aus der Schnittdarstellung von Fig. 3 ersichtlich ist. Dadurch ist zwischen der Außenseite der Hülse 36 und der Innenseite des Gehäuses 24 ein Ringraum 58 geschaffen, in dem eine Torsionsfeder 57 aufgenommen ist.

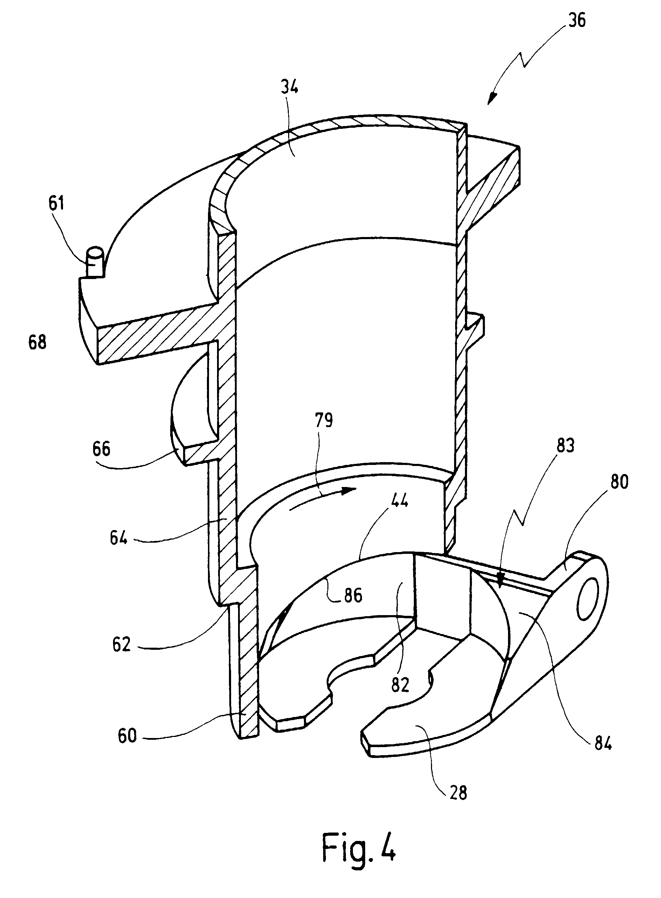

Die Torsionsfeder 57 ist schraubenlinienförmig um die Außenseite der Hülse 36 gewunden, ein Ende 59 ist in der Wand 53 des Gehäuses 24 verankert, das andere Ende 61 steht mit einem Flansch 68 der Hülse 36 in Eingriff (siehe auch Fig. 4).

Die Hülse 36 weist, wie das insbesondere aus den Darstellungen von Fig. 3 und 4 ersichtlich ist, einen unteren Endabschnitt 60 auf, der über eine Schulter 62 in einen mittleren etwas durchmessergrößeren Abschnitt 64 übergeht.

Von der Außenseite des Abschnittes 64 springen axial beabstandet zwei Ringflansche 66 und 68 vor, zwischen denen die Torsionsfeder 57 aufgenommen ist, wie das aus der Schnittdarstellung von Fig. 3 zu entnehmen ist.

Wie aus der aufgeschnittenen Darstellung von Fig. 2 zu entnehmen, ist von oben in die Hülse 36 ein Einsatzstück 70 eingeschoben, das den eigentlichen Instrumentenkanal 16 umgrenzt. Ein unteres Ende des Einsatzstück 70 stellt einen Ventilsitz 74 (siehe Fig. 1 und 2) für die Dichthalbkugel 30 des Klappventils 26 dar. Im endmontierten Zustand ist auf das Einsatzstück 70 noch eine Kappe 72 aufgesetzt.

Im geschlossenen Zustand des Klappventils 26, wie er in der Schnittdarstellung von Fig. 3 ersichtlich ist, ist die Klappe 28 in den Instrumentenkanal 16 eingeschwenkt und liegt am zuvor erwähnten Ventilsitz 74 dichtend an. Die Klappe 28 liegt dabei im Innern des unteren Endabschnitts 60 der Hülse 36. Die beiden diametral gegenüberliegenden Anlaufschrägen 42 und 44 stehen dabei mit einem Steuerabschnitt 83 der Klappe 28 des Klappventils 26 in Berührung.

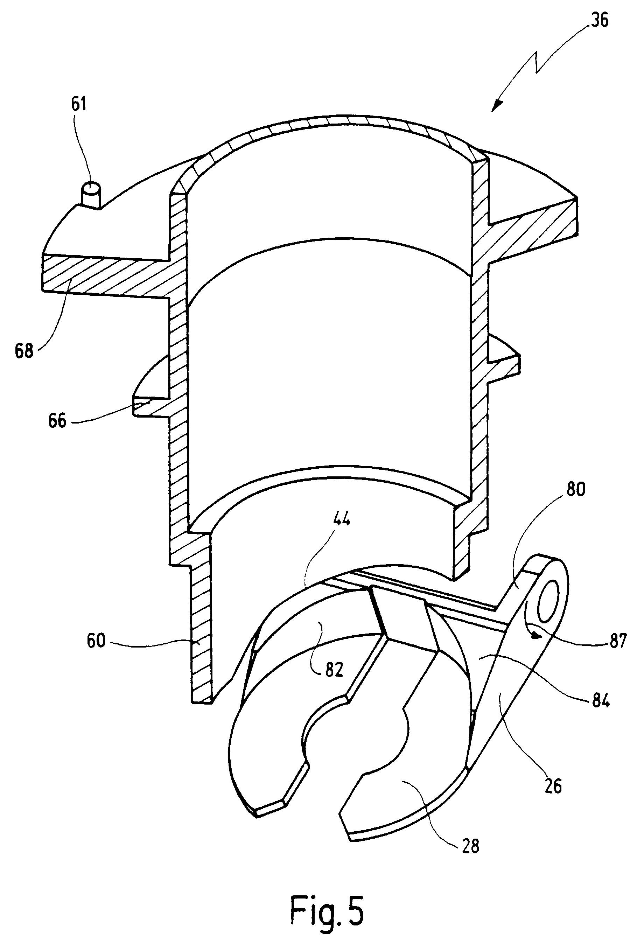

Wie aus der vergrößerten Darstellung von Fig. 4 zu entnehmen, besteht die Klappe 28 aus einem flächigen Basisteil, in den die Dichthalbkugel 30 eingedrückt ist, der Übersichtlichkeit halber ist diese Dichthalbkugel 30 in den Darstellungen von Fig. 4 und 5 weggelassen. Etwa tangential springen gabelartig zwei Laschen 80 und 82 von dem hier nicht näher bezeichneten Basisteil vor, die mit entsprechenden fluchtenden Öffnungen versehen sind, in die der Achszapfen 32 eingeschoben wird. Gleichzeitig wird der Achszapfen 32 durch entsprechende fluchtende Öffnungen von Montagelaschen an der Unterseite des Gehäuses 24 geschoben, somit die Klappe 28 schwenkbar befestigt.

Die in der Darstellung von Fig. 4 und 5 oberen Kanten der Laschen 80 und 82 gehen über Schrägen 84 bzw. 86 in das Basisteil der Klappe 28 über. Dieser Bereich, also der Übergangsbereich von den oberen Kanten der Laschen 80 und 82 und die Schrägen 84 und 86 bilden den Steuerabschnitt 83, der mit den Anlaufschrägen 42 und 44 in Berührung steht.

In geschlossenem Zustand, wie dies in Fig. 3 dargestellt ist, steht die Lasche 80 etwa im Übergangsbereich zwischen deren Oberkante und der Schräge 34 mit der Anlaufschräge 42 in Berührung. Entsprechendes gilt für die Lasche 82 und die Anlaufschräge 44. Wird nun die Hülse 36 über eines der Griffelemente 50 oder 51 so verdreht, wie dies in Fig. 3a durch einen Pfeil 77 angedeutet ist, drückt die Anlaufschräge 42 die Klappe 28 nach unten weg, dabei läuft der als Steuernocken ausgebildete Übergangsbereich zwischen Oberkante der Lasche 38 und der Schräge 84 längs der Anlaufschräge 24 nach unten.

Ein weiteres Verdrehen, wie das bei dem Übergang von Fig. 3a nach 3b durch einen Pfeil 78 angedeutet ist, bewirkt nun ein vollständiges Öffnen der Klappe 28, d.h. diese ist vollständig aus dem Instrumentenkanal 16 herausgeschwenkt.

In den vergrößerten ausschnittsweisen Darstellungen von Fig. 4 und 5 ist dieser Bewegungsablauf nochmals dargestellt, wobei hier nun die umgekehrte Drehrichtung dargestellt ist.

Das heißt, wie das aus Fig. 4 zu entnehmen ist, wird die Hülse 36 in Richtung eines Pfeiles 79 bewegt, d.h. genau umgekehrt wie in Richtung längs des Pfeiles 77 von Fig. 3a. In diesem Fall tritt dann die Anlaufschräge 44 mit dem entsprechenden Steuerabschnitt der Lasche 82 in Eingriff und öffnet bzw. verschwenkt die Klappe 28 im gleichen Sinne wie bei der Abfolge der Fig. 3, 3a und 3b.

Somit wird die Klappe 28 in beiden Drehrichtungen der Hülse 36 geöffnet.

Dadurch, daß die Hülse 36 mit einem Ende 61 der Torsionsfeder 57 in Eingriff steht, wie das in Fig. 4 angedeutet ist, wird bei Verdrehen der Hülse 36 die Torsionsfeder 57 gespannt, so daß diese die Tendenz hat, die Hülse 36 wieder in die Ausgangsstellung (Fig. 3 bzw. Fig. 4) zurückzudrehen.

Würden also bspw. aus der Darstellung von Fig. 3b die Griffelemente 50, 51 losgelassen, würde die Torsionsfeder 57 die Hülse 36 wieder zurückdrehen, und aufgrund der Kraft der Feder 33 würde auch gleichzeitig wieder das Klappventil 26 geschlossen.

## Patentansprüche

1. Rohrschaftinstrument, insbesondere Trokar (12), mit einem Instrumentenkanal (16), der durch ein Klappventil (26) verschließbar ist, sowie mit einem Betätigungselement, um das Klappventil (26) gegen die Kraft einer Schließfeder (33) zu öffnen, dadurch gekennzeichnet, daß das Betätigungselement als ein den Instrumentenkanal (16) zumindest teilweise umrundendes, um die Instrumentenkanalachse (18) verdrehbares Element (34) ausgebildet ist, das mit einer umfänglichen, in einer Richtung parallel zur Instrumentenkanalachse (18) ansteigenden Anlaufschräge (42, 44) versehen ist, auf der ein Steuerabschnitt (83) des Klappventils (26) ruht, so daß ein Verdrehen des verdrehbaren Elementes (34) ein Öffnen bzw. ein Schließen des Klappventils (26) bewirkt.

2. Rohrschaftinstrument nach Anspruch 1, dadurch gekennzeichnet, daß das verdrehbare Element (34) zwei gegenüberliegende gleichsinnige Anlaufschrägen (42, 44) aufweist, so daß ein Drehen des verdrehbaren Elementes (34) in die eine (77, 78) oder in die entgegengesetzte Richtung (79) jeweils dieselbe Bewegung des Klappventils (26) verursacht.

3. Rohrschaftinstrument nach Anspruch 1 oder 2, dadurch gekennzeichnet, daß das verdrehbare Element (34) als Hülse (36) ausgebildet ist, deren dem Klappventil (26) zugewandtes Ende (38) schräg abgeschnitten ist, wobei die resultierenden Schnittkanten die Anlaufschrägen (42, 40) bilden.

4. Rohrschaftinstrument nach Anspruch 3, dadurch gekennzeichnet, daß das Ende (38) des verdrehbaren Elementes (34) etwa über einen halben Umfang schräg abgeschnitten ist.

5. Rohrschaftinstrument nach Anspruch 3 oder 4, dadurch gekennzeichnet, daß der Schnittwinkel etwa unter 45° zur Instrumentenkanalachse (18) verläuft.

6. Rohrschaftinstrument nach einem der Ansprüche 1 bis 5, dadurch gekennzeichnet, daß das verdrehbare Element (34) mit zumindest einem radial abstehenden Griffelement (50, 51) versehen ist.

7. Rohrschaftinstrument nach Anspruch 6, dadurch gekennzeichnet, daß zwei diametral gegenüberliegende, radial abstehende Griffelemente (50, 51) vorgesehen sind.

8. Rohrschaftinstrument nach einem der Ansprüche 1 bis 7, dadurch gekennzeichnet, daß das verdrehbare Element (34) gegen die Kraft einer Torsionsfeder (57) verdrehbar ist, die das verdrehbare Element (34) in eine Drehstellung dreht, in der das Klappventil (26) geschlossen ist.

9. Rohrschaftinstrument nach Anspruch 8, dadurch gekennzeichnet, daß die Torsionsfeder (57) in einem Ringraum (58) zwischen Außenseite des verdrehbaren Elementes (54) und einer Innenseite eines das Element (34) umschließenden Gehäuses (24) angeordnet ist.

## Claims

1. Tubular shaft instrument, in particular a trocar (12), having an instrument channel (16) which can be closed off by a flap valve (26), and having an actuation element in order to open the flap valve (26) against the force of a closing spring (33), characterized in that the actuation element is configured as an element (34) which at least partly surrounds the instrument channel (16) and can rotate about the instrument channel axis (18), and which is equipped with a circumferential cam bevel (42, 44), rising in a direction parallel to the instrument channel axis (18), on which a control section (83) of the flap valve (26) rests, so that a rotation of the rotatable element (34) effects opening or closing of the flap valve (26).

2. Tubular shaft instrument according to claim 1, characterized in that the rotatable element (34) has two cam bevels (42, 44) opposite one another and running in the same direction, so that a rotation of the rotatable element (34) in one direction (77, 78) or the opposite direction (79) causes the same movement of the flap valve (26) in each case.

3. Tubular shaft instrument according to claims 1 or 2, characterized in that the rotatable element (34) is configured as a sleeve (36) whose end (38) facing the flap valve (26) is cut off obliquely, the resulting cut edges constituting the cam bevels (42, 40).

4. Tubular shaft instrument according to claim 3, characterized in that the end (38) of the rotatable element (34) is cut off obliquely over approximately half a circumference.

5. Tubular shaft instrument according to claims 3 or 4, characterized in that the cut angle extends at approximately 45 degrees to the instrument channel axis (18).

6. Tubular shaft instrument according to any of claims 1 through 5, characterized in that the rotatable element (34) is equipped with at least one radially projecting grip element (50, 51).

7. Tubular shaft instrument according to claim 6, characterized in that two diametrically opposite radially projecting grip elements (50, 51) are provided.

8. Tubular shaft instrument according to any of claims 1 through 7, characterized in that the rotatable element (34) is rotatable against the force of a torsion spring (57) which rotates the rotatable element (34) into a rotary position in which the flap valve (26) is closed.

9. Tubular shaft instrument according to claim 8, characterized in that the torsion spring (57) is arranged in an annular space (58) between the outer side of the rotatable element (54) and an inner side of a housing (24) surrounding the element (34).

## Revendications

1. Instrument à tige tubulaire, notamment trocart (12), comportant un canal d'instrument (16), qui peut être fermé par un clapet (26), ainsi qu'un élément d'actionnement, pour ouvrir le clapet (26) à l'encontre de la force d'un ressort de fermeture (33), caractérisé en ce que l'élément d'actionnement est réalisé en tant qu'élément (34) qui entoure au moins partiellement le canal d'instrument (16), qui peut tourner autour de l'axe (18) du canal d'instrument et qui est pourvu d'une surface d'arrêt (42, 44) montant dans une direction parallèle à l'axe (18) du canal d'instrument, et sur laquelle repose un segment de commande (83) du clapet (26), ce qui fait qu'une rotation, de l'élément tournant (34) provoque une ouverture ou une fermeture du clapet (26).

2. Instrument à tige tubulaire selon la revendication 1, caractérisé en ce que l'élément tournant (34) présente deux surfaces d'arrêt (42, 44) en vis-à-vis et de même sens, ce qui fait qu'une rotation de l'élément tournant (34) dans un sens (77, 78) ou dans le sens opposé (79) provoque toujours le même mouvement du clapet (26).

3. Instrument à tige tubulaire selon la revendication 1 ou 2, caractérisé en ce que l'élément tournant (34) est réalisé sous la forme d'une canule (36) dont l'extrémité (38) tournée vers le clapet (26) est coupée en biais, les bords de coupe résultants formant les surfaces d'arrêt (42, 40).

4. Instrument à tige tubulaire selon la revendication 3, caractérisé en ce que l'extrémité (38) de l'élément tournant (34) est coupée en biais sur un demi-périmètre.

5. Instrument à tige tubulaire selon la revendication 3 ou 4, caractérisé en ce que l'angle de coupe s'étend à peu près sous 45° par rapport à l'axe (18) du canal d'instrument.

6. Instrument à tige tubulaire selon l'une des revendications 1 à 5, caractérisé en ce que l'élément tournant (34) est pourvu d'au moins un élément formant poignée (50, 51) qui dépasse radialement.

7. Instrument à tige tubulaire selon la revendication 6, caractérisé en ce que sont prévus deux éléments formant poignées (50, 51) qui sont diamétralement opposés et qui dépassent radialement.

8. Instrument à tige tubulaire selon l'une des revendications 1 à 7, caractérisé en ce que l'élément tournant (34) peut tourner à l'encontre de la force d'un ressort de torsion (57) qui fait tourner l'élément tournant (34) dans une position de rotation dans laquelle le clapet (26) est fermé.

9. Instrument à tige tubulaire selon la revendication 8, caractérisé en ce que le ressort de torsion (57) est disposé dans un espace annulaire (58) entre le côté extérieur de l'élément tournant (54) et un côté intérieur d'un boîtier (24) entourant l'élément (34).
